# EUROPÄISCHE PATENTSCHRIFT

(11) **EP 3 574 863 B1**
(45) Veröffentlichungstag und Bekanntmachung des Hinweises auf die Patenterteilung: **17.02.2021**
(21) Anmeldenummer: 19167812.7
(22) Anmeldetag: 08.04.2019
(51) Int. Cl.: A61B 90/50, A61B 34/00, A61B 18/00, H01H 9/00, H01H 9/04

(54) **AUTOKLAVIERBARER HANDGRIFF**
AUTOCLAVABLE HANDLE
POIGNÉE POUVANT ÊTRE AUTOCLAVÉ

(30) Priorität: 28.05.2018 DE 102018004244
(43) Veröffentlichungstag der Anmeldung: 04.12.2019
(73) Patentinhaber: Karl Storz SE & Co. KG, 78532 Tuttlingen (DE)
(72) Erfinder: Stefan, Jochen, 78532 Tuttlingen (DE); Kramer, Claus, 78532 Tuttlingen (DE); Grüner, Sven, 78532 Tuttlingen (DE); Fan, Chunman, 78532 Tuttlingen (DE); Behrendt, Kay, 78532 Tuttlingen (DE); Bayer, Andreas, 78532 Tuttlingen (DE)
(74) Vertreter: Hofmeister, Frank

(56) Entgegenhaltungen:
- EP-A2- 2 641 550
- DE-A1-102004 041 871
- DE-U1-202006 018 987
- US-A- 5 712 543
- US-A- 6 059 806
- US-A1- 2003 214 816
- US-A1- 2006 100 485
- US-A1- 2016 310 134

## Beschreibung

Die Erfindung betrifft einen autoklavierbarer Handgriff mit einem Handgriffgehäuse und mit mindestens einem im Handgriffgehäuse gelagerten Schaltelement, wobei das mindestens eine Schaltelement federelastisch in das Handgriffgehäuse eindrückbar ist, wobei das Schaltelement über mindestens einen an der Unterseite des Schaltelements angeordneten Steg im Handgriffgehäuse gelagert ist und das Schaltelement gegenüber dem Handgriffgehäuse über ein umlaufendes Dichtungselement abgedichtet ist.

Handgriffe mit Schaltelementen, beispielsweise elektromechanischen Tastern, sind als gerätegebundene Schalteinrichtungen oder auch als Fernbedienungen in den verschiedensten Ausführungsformen aus dem privaten und beruflichen Bereich bekannt. In der Medizintechnik stellen diese Handgriffe mit den elektrischen Schaltelementen ein wichtiges Hilfsmittel in der Schnittstelle zwischen dem Benutzer und dem angeschlossenen Gerät dar. In Operationsräumen dienen diese im sterilen Bereich bedienbaren Handgriffe einerseits zur Bedienung von in einem nicht sterilen Bereich angeordneten Geräten oder andererseits von im sterilen Bereich angeordneten Geräten, wie beispielsweise Haltearmen oder auch Operationsrobotern, die, da sie nicht als ganzes autoklavierbar sind, während der Operation unter sterilen Tüchern und Planen abgedeckt sind.

Wenn ein Bedienungshandgriff nicht als Einmalartikel verwendet werden soll, muss dieser nach jedem Gebrauch sterilisiert werden. Die übliche Form der Sterilisation ist das Autoklavieren in einem Autoklaven.

Bei der auch Dampfsterilisation genannten Autoklavierung wird in der Praxis üblicherweise das fraktionierte Vakuumverfahren verwendet. Hierbei wird in der Entlüftungsphase im Autoklaven ein Vakuum erzeugt. Anschließend wird Wasserdampf in die Sterilisierkammer des Autoklaven eingelassen und in der Sterilisierphase auf 134°C erhitzt und bei einem Druck von 3 bar über einen Zeitraum von mindestens 5 Minuten die im Autoklaven befindlichen zu sterilisierenden Geräte sterilisiert. In der abschließenden Trocknungsphase wird der Wasserdampf abgelassen und unter Anlegen eines erneuten Vakuums das Sterilisiergut getrocknet. Diese vorgenannten Verfahrensschritte der Autoklavierung werden in der Regel mehrfach wiederholt.

Dieser voranstehend beschriebene Autoklavierprozess stellt für die zu sterilisierenden Geräte eine außerordentliche Beanspruchung für das Material, die Dichtungen und im Falle der Handgriffe auch für die Elektronik dar.

Aus der Praxis sind mit elektrischen Schaltelementen versehene Handgriffe bekannt, die für die Sterilisation geöffnet und zerlegt werden müssen. Diese zerlegbaren Handgriffe weisen aufgrund der vorhandenen Fügestellen den Nachteil auf, dass im Betrieb Verschmutzungen und Feuchtigkeit in den Handgriff eindringen können. Weiterhin ist das Demontieren und anschließende erneute Zusammenfügen der Handgriffe mit einem hohen Zeitaufwand und somit auch hohen Arbeitskosten verbunden.

Ein gattungsgemäßer Handgriff ist beispielsweise aus der US 5 712 543 A bekannt. Bei diesem bekannten Handgriff sind mehrere Schaltelemente dicht beieinander in einer Vertiefung im Griffgehäuse angeordnet, wodurch eine individuelle Ansteuerung der einzelnen Schaltelemente erschwert wird.

Aus der US 6 059 806 A ist weiterhin eine chirurgische Handbohrmaschine bekannt, deren Gehäuse autoklavierbar ist, jedoch alle elektrischen Elemente als kompakter Energieblock vor dem Autoklavieren aus dem Handgriff entfernt werden müssen. Spezielle Abdichtungen zum Schutz elektrischer Schaltkontakte sind bei dieser bekannten Ausgestaltungsform nicht erforderlich.

Weiterhin sind aus der DE 10 2004 041 871 B4 eine autoklavierbare Fernbedienung sowie ein Verfahren zu deren Herstellung bekannt. Bei dieser bekannten Fernbedienung, wird in einem ersten Arbeitsschritt die mit den elektrischen Schaltelementen versehene Platine mit Silikon so umgossen, dass nur die Schalter bzw. Taster aus der Silikon-Vergussmasse des Basisteils herausragen. Die Abdichtung der Schalter bzw. Taster erfolgt in einem zweiten Arbeitsschritt über eine aus einem autoklavierbaren Material bestehende Abdeckung, die mit der Silikon-Vergussmasse des Basisteils verbunden, beispielsweise verklebt.

Das Umgießen der mit den elektrischen Schaltelementen bestückten Platine mit Silikon stellt zwar eine sehr gute Abdichtung dieses Basisteils dar, jedoch weist auch diese bekannte autoklavierbare Fernbedienung eine Fügestelle, nämlich die zwischen dem in Silikon eingegossenen Basisteil und der Abdeckung auf. Diese verklebte Fügestelle weist zwei potentielle Problempunkte auf, nämlich einerseits die ordnungsgemäße und vollständige Ausbildung der vollflächigen Verklebung und andererseits die Alterung der Klebenaht insbesondere im Laufe der mehrfachen Autoklavierung.

Davon ausgehend liegt der Erfindung die **Aufgabe** zugrunde, einen autoklavierbaren Handgriff der eingangs genannten Art zu schaffen, der gegenüber den Einflüssen der maschinellen Reinigung und Dampfsterilisation dauerhaft beständig ausgebildet ist und eine hohe Bedienungssicherheit aufweist.

Die Lösung dieser Aufgabenstellung ist erfindungsgemäß dadurch gekennzeichnet, dass das Handgriffgehäuse mehrere in jeweils einer zugehörigen wannenförmigen Vertiefung des Handgriffgehäuses gelagerte Schaltelemente aufweist, wobei in jeder Vertiefung eine Öffnung zum Durchtritt des mindestens einen Stegs ausgebildet ist und wobei für jeden Steg eine separate Öffnung in der wannenförmigen Vertiefung ausgebildet ist und die Stege aller Schaltelemente auf einer gemeinsamen Achse im Handgriffgehäuse gelagert sind.

Durch die Verwendung des federelastisch in das Handgriffgehäuse eindrückbaren Schaltelemets mit der umlaufenden Dichtung aus einem autoklavierbaren Material, die den Spalt zwischen dem beweglichen Schaltelement und dem starren Handgriffgehäuse abdichtend überbrückt, wird ein Handgriff geschaffen, der bei einfaeher und sicherer Handhabung ohne Fügestellen im Gehäuse auskommt und daher eine zuverlässige Reinigung im Autoklaven gewährleistet.

Die wannenförmige Vertiefung zur Aufnahme des eindrückbaren Schaltelements lässt sich einfach über das umlaufende Dichtungselement abdichten, das den Spalt zwischen dem beweglichen Schaltelement und dem starren Handgriffgehäuse abdichtend überbrückt.

Die Verwendung mehrerer Schaltelemente in einem Handgriffgehäuse kann entweder einerseits dazu verwendet werden, mehrere Schaltfunktionen mit einem Handgriff zu ermöglichen, oder aber andererseits dazu dienen, die Bedienungssicherheit dadurch zu erhöhen, dass ein Schaltsignal erst dann erzeugt wird, wenn zwei oder mehr Schaltelemente gleichzeitig betätigt werden.

Gemäß einer bevorzugten Ausführungsform der Erfindung wird vorgeschlagen, dass in dem mindestens einen Steg ein in Längsrichtung des Stegs verlaufendes Langloch ausgebildet ist und der Steg über eine das Langloch durchragende Achse im Handgriffgehäuse gelagert ist, wobei am Schaltelement ein Federelement derart gelagert ist, dass das Federelement das Schaltelement nach außen aus dem Handgriffgehäuse drückt, so dass sich ein Arbeitsweg des Schaltelements aus der Länge des Langlochs ergibt. Diese Art der Lagerung des mindestens einen Schaltelements gewährleistet einerseits eine sichere und unverlierbare Lagerung des Schaltelements im Handgriffgehäuse und garantiert andererseits gleichzeitig einen definierten Arbeitshub für das in das Handgriffgehäuse eindrückbare Schaltelement.

Um das federelastische Eindrücken der Schaltelemente in das Handgriffgehäuse zu ermöglichen, wird erfindungsgemäß vorgeschlagen, dass das Federelement mit einem Ende an der Unterseite des Schaltelements anliegt und sich mit dem anderen Ende am Boden der wannenförmigen Vertiefung des Handgriffgehäuses abstützt. Diese Anordnung des Federelements, wobei das Federelement vorteilhafterweise in der Mitte des jeweiligen Schaltelements angeordnet ist, ermöglicht bei einfacher Montage eine leichtgängige Bedienbarkeit des jeweiligen Schaltelements.

Bei einer erfindungsgemäß bevorzugten Ausführungsform zur Ausbildung der Schaltelemente ist das jedes Schaltelement über zwei voneinander beabstandete Stege im Handgriffgehäuse gelagert, wobei beide Stege auf einer gemeinsamen Achse im Handgriffgehäuse gelagert sind und für jeden Steg eine separate Öffnung in der wannenförmigen Vertiefung des Handgriffgehäuses ausgebildet ist. Die Verwendung von zwei voneinander beabstandeten Stegen, wobei zwischen den beiden Stegen das Federelement gelagert ist, stabilisiert einerseits die Lagerung des Schaltelements auf dem Federelement und ermöglicht es andererseits, dass das Schaltelement auch durch ein wippendes Verkippen betätigt werden kann.

Um eine zuverlässige Abdichtung zwischen dem Handgriffgehäuse einerseits und dem eindrückbaren Schaltelement andererseits zu gewährleisten, wird mit der Erfindung vorgeschlagen, dass das jeweilige umlaufende Dichtungselement, über das jedes Schaltelement gegenüber dem Handgriffgehäuse abgedichtet ist, sowohl auf Seiten des Handgriffgehäuses als auch auf Seiten des jeweiligen Schaltelements klemmend gelagert ist. Eine beidseitig klemmende Lagerung des Dichtungselements garantiert einen sicheren und ortsfesten Halt des Dichtungselements auch beim Betätigen des zugehörigen Schaltelements.

Gemäß einer praktischen Ausführungsform zur Ausbildung des Dichtungselements wird mit der Erfindung vorgeschlagen, dass jedes Dichtungselement zwei in Radialrichtung voneinander beabstandete wulstförmige Klemmbereiche aufweist, die über einen elastischen Verbindungssteg miteinander verbunden sind. Der elastische Verbindungssteg gewährleistet das federelastische Eindrücken der Schaltelemente in das Handgriffgehäuse.

Jedes Schaltelement besteht erfindungsgemäß vorteilhafterweise aus einer oberen Griffplatte und einer unteren Schraubplatte, wobei das umlaufende Dichtungselement klemmend in einer Dichtungsaufnahme zwischen der Griffplatte und der Schraubplatte festgelegt ist. Die Verwendung des zweiteiligen Aufbaus mit der unteren Schraubplatte hat den Vorteil, dass das Dichtungselement auf einfache Art und Weise klemmend an dem jeweilige Schaltelement festlegbar ist und alle notwendigen Schraubverbindungen auf der Unterseite des Schaltelements angeordnet sind, die durch das umlaufende Dichtungselement gegenüber der Umgebung abgedichtet ist. Die üblichen schwer zu reinigenden Stellen, wie beispielsweise Schraubenlöcher, sind somit bei dieser erfindungsgemäßen Ausgestaltung konstruktionsbedingt bereits so angeordnet, dass diese von außen nicht zugänglich sind.

Das klemmende Festlegen des Dichtungselements auf Seiten des Handgriffgehäuses erfolgt erfindungsgemäß dadurch, dass im Handgriffgehäuse umlaufend um jede wannenförmige Vertiefung zur Aufnahme eines Schaltelements eine Nut zur Aufnahme des jeweiligen Dichtungselements ausgebildet ist, wobei die jeweilige Nut bei eingelegtem Dichtungselement über eine am Handgriffgehäuse festlegbare Klemmplatte abdeckbar ist. Die Klemmplatte ihrerseits ist vorteilhafterweise über eine Rastverbindung am Handgriffgehäuse festlegbar.

Gemäß einer bevorzugten Ausführungsform zur Ausbildung eines erfindungsgemäßen Handgriffs wird vorgeschlagen, dass das Handgriffgehäuse im Querschnitt dreieckig ausgebildet ist und, dass in jeder der drei Seitenflächen des Handgriffgehäuses jeweils eine wannenförmige Vertiefung ausgebildet ist, in der ein Schaltelement federelastisch in das Handgriffgehäuse eindrückbar gelagert ist. Bei der Verwendung des Handgriffs an einem medizinischen Haltearm dient der Handgriff dazu, über die Schaltelemente die Gelenke des Haltearms zu lösen, wenn dieser neu positioniert werden soll. In dem Moment, in dem der Bediener die Schaltelemente betätigt und die Gelenke des Haltearms löst, muss der Bediener das gesamte Gewicht des Haltearms über den Halt am Handgriff abfangen. Die dreieckige Form des Handgriffgehäuses bietet einen sicheren Zugriff, und verhindert auch ein Verdrehen des Handgriffs in der Hand des Bedieners, wie dies bei rund geformten Handgriffen passieren kann.

Weiterhin wird mit der Erfindung vorgeschlagen, dass unterhalb jedes Schaltelements mindestens ein elektrischer Kontakt, beispielsweise ein Taster, so angeordnet ist, dass das Schaltelement den zugehörigen mindestens einen elektrischen Kontakt beim Eindrücken in das Handgriffgehäuse aktiviert. Vorteilhafterweise ist der mindestens eine elektrische Kontakt in der wannenförmigen Vertiefung des Handgriffgehäuses angeordnet.

Gemäß einer praktischen Ausführungsform der Erfindung wird vorgeschlagen, dass unterhalb jedes Schaltelements zwei voneinander beabstandete elektrische Kontakte angeordnet sind, wobei die beiden elektrischen Kontakte eines jeden Schaltelements in Parallelschaltung geschaltet sind. Die Verwendung von zwei elektrischen Kontakten ermöglicht auch eine wippende Betätigung des Schaltelements über das Federelement. Aufgrund der Parallelschaltung der beiden elektrischen Kontakte ist es für das Erzeugen eines Schaltsignals egal, ob der vordere oder der hintere Kontakt aktiviert wird.

Weiterhin betrifft die Erfindung ein Verfahren zum Betätigen der Schaltelemente eines autoklavierbaren Handgriffs nach einem der Ansprüche 1 bis 13, wobei im Handgriffgehäuse mindestens zwei federelastisch in das Handgriffgehäuse eindrückbare Schaltelemente gelagert sind. Um die Betriebssicherheit des Handgriffs zu erhöhen und insbesondere ein versehentliches Betätigen eines Schaltelements auszuschließen, wird mit der Erfindung vorgeschlagen, dass alle im Handgriffgehäuse angeordneten Schaltelemente zur Erzeugung eines Schaltsignals gleichzeitig betätigt werden müssen. Das versehentliche Betätigen nicht aller Schaltelemente des jeweiligen Handgriffs bleibt somit unschädlich.

Die Erzeugung eines Schaltsignals nur dann, wenn alle Schaltelemente gleichzeitig betätigt werden, wird erfindungsgemäß dadurch erzielt, dass alle im Handgriffgehäuse angeordneten Schaltelemente in Reihe geschaltet sind.

Zur Überwachung der einzelnen Schaltelemente gegenüber einem Kurzschluss-Defekt wird erfindungsgemäß vorgeschlagen, dass jedes Schaltelement mittels einer nach dem Ruhestromprinzip arbeitenden Sicherheitsschaltung überwacht wird.

Schließlich wird mit der Erfindung vorgeschlagen, dass alle Schaltelemente eines Handgriffs mittels eines Zeitmessglieds auf das Vorliegen eines Kurzschluss-Defekts derart überwacht werden, dass beim Ausfall eines Schaltelements alle anderen Schaltelemente dieses Handgriffs deaktiviert werden. Im Falle des Einsatzes an einem medizinischen Haltearm werden bei der Deaktivierung der Schaltelemente alle Gelenke des Haltearms in der die Gelenke sperrenden Stellung gehalten.

Weitere Merkmale und Vorteile der Erfindung ergeben sich anhand der zugehörigen Zeichnungen, in denen ein Ausführungsbeispiel eines erfindungsgemäßen autoklavierbaren Handgriffs nur beispielhaft dargestellt ist, ohne die Erfindung auf dieses Ausführungsbeispiel zu beschränken. In den Zeichnungen zeigt:
- Fig. 1: eine schematische Darstellung eines medizinischen Tragarms;
- Fig. 2: eine vergrößerte perspektivische Darstellung des Details II, einen erfindungsgemäßen autoklavierbaren Handgriff darstellend;
- Fig. 3: eine perspektivische Ansicht des Handgriffgehäuses des Handgriffs gemäß Fig. 2;
- Fig. 4: eine perspektivische Ansicht gemäß Fig. 2, jedoch den Handgriff ohne das Handgriffgehäuse darstellend;
- Fig. 5: eine vergrößerte Darstellung des Details V gemäß Fig. 4;
- Fig. 6: einen Schnitt entlang der Linie VI-VI gemäß Fig. 4;
- Fig. 7: einen Längsschnitt durch das Detail VII gemäß Fig. 2;
- Fig. 8: eine vergrößerte Seitenansicht der Griffplatte eines Schaltelements des Handgriffs gemäß Fig. 2;
- Fig. 9: eine vergrößerte perspektivische Ansicht der Schraubplatte eines Schaltelements des Handgriffs gemäß Fig. 2;
- Fig. 10: eine vergrößerte perspektivische Ansicht eines Dichtungselements des Handgriffs gemäß Fig. 2;
- Fig. 11: eine vergrößerte perspektivische Ansicht einer Klemmplatte des Handgriffs gemäß Fig. 2 und
- Fig. 12: ein schematisches Blockschaltbild der Schaltelemente des Handgriffs.

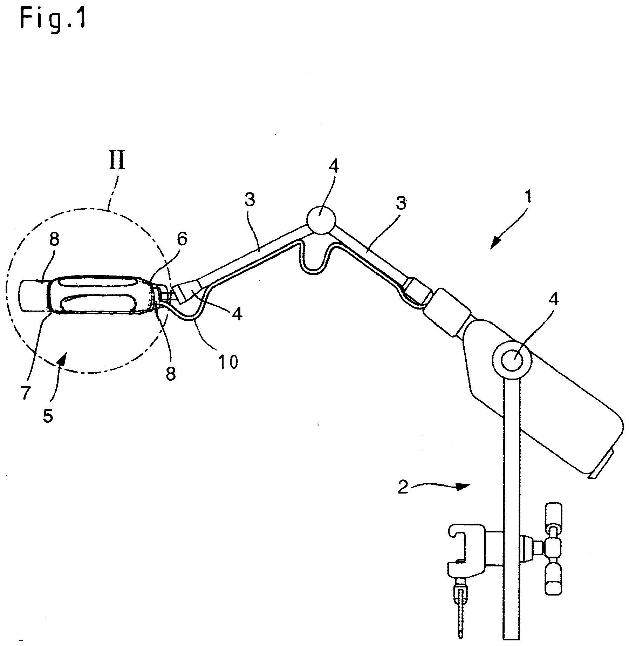

Die Abbildung Fig. 1 zeigt schematisch einen medizinischen Haltearm 1, der aus einem Basisteil 2 sowie mehreren Armsegmenten 3 besteht, die über Gelenke 4 miteinander sowie mit dem Basisteil 2 verbunden sind. Derartige Haltearme 1 dienen dazu, bei Operationen Instrumente oder allgemein Objekte im Operationsfeld, meist oberhalb des Patienten, zu halten, um so den Operateur zu entlasten. Ansonsten müsste der Operateur oder ein Assistent die Objekte selbst in der vorgesehenen Stellung relativ zum Patienten halten.

Zur Änderung der Halteposition des Haltearms 1 müssen ein oder mehrere Gelenke 4 des Haltearms 1 gelöst werden und nach der Repositionierung des Haltearms 1 wieder blockiert werden, um den Haltearm 1 in der neuen Position zu fixieren. Das Lösen des Haltesystems des Haltearms 1 sowie das erneute blockieren erfolgt über einen Handgriff 5 in dem verschiedene elektrische und/oder elektromechanische Schaltelemente, wie insbesondere Taster, angeordnet sind.

Da die Haltearme 1 im sterilen Bereich des Operationssaals angeordnet sind, aber nicht als Ganzes autoklavierbar sind, wird zumindest das nicht demontierbare Basisteil 2 des Haltearms 1 während der Operation unter sterilen Tüchern und Planen abgedeckt. Nur die autoklavierbaren Bereiche, wie eben beispielsweise die Handgriffe 5, bleiben frei zugänglich und müssen, wenn sie nicht als Einmalartikel ausgebildet sind, nach jedem Gebrauch sterilisiert werden. Die übliche Form der Sterilisation ist das Autoklavieren in einem Autoklaven.

Damit der Bediener des Handgriffs 5 die Last des Tragarms 1 beim Entsperren der Gelenke 4 direkt auffangen kann, ist der Handgriff 5 zum Bedienen des Tragarms 1 vorteilhafterweise, wie in Fig. 1 dargestellt, so mit dem Tragarm 1 gekoppelt, dass dieser im Betrieb fest mit dem Tragarm 1 verbunden ist, aber zum Autoklavieren vom Tragarm 1 lösbar ist.

Bei dem in Fig. 1 dargestellten Haltearm 1 ist der Handgriff 5 so angeordnet, dass dieser in Längsrichtung des Handgriffs 5 betrachtet mit seinem proximalen Ende 6 mit dem Tragarm 1 gekoppelt ist und ein relativ zum Patienten zu positionierendes medizinisches Instrument am in Längsrichtung des Handgriffs 2 betrachtet distalen Ende 7 des Handgriffs 5 festlegbar ist.

Das Verbinden des Handgriffs 5 mit dem Tragarm 1 bzw. einem anzuschließenden medizinischen Instrument erfolgt über Kopplungsanschlüsse 8, die am proximalen Ende 6 und distalen Ende 7 eines Handgriffgehäuses 9 des Handgriffs 5 ausgebildet sind. Bei diesen Kopplungsanschlüsse 8 handelt es sich um von außen an das Handgriffgehäuse 9 angeformte Bauteile, die über keine Verbindung zum Innenraum des Handgriffgehäuses 9 verfügen und somit auch keine Naht- oder Fügestelle zum Bereich des Handgriffgehäuses 8 aufweisen.

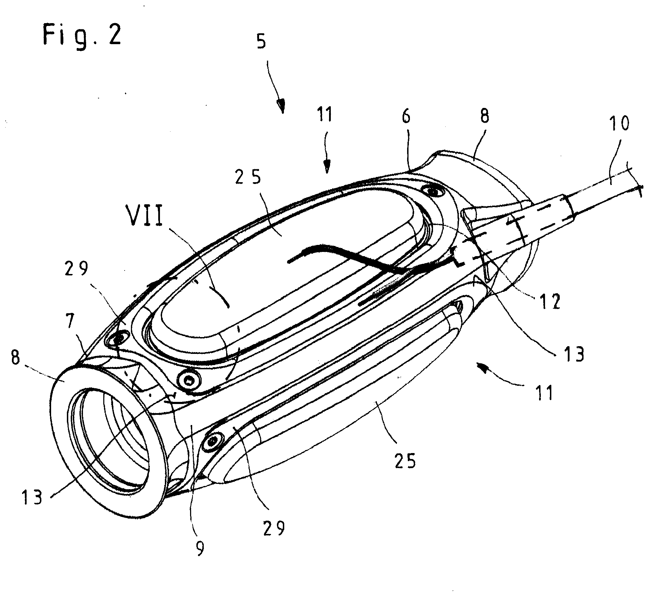

Bei der in Fig. 1 und 2 dargestellten Ausführungsform ist der Handgriff 5 zur Energieversorgung sowie zum Übertragen der Schaltsignale mit einem elektrischen Kabel 10 versehen. Alternativ ist es jedoch auch möglich, den Handgriff 5 kabellos auszubilden, wobei die Schaltsignale vom Handgriff 5 zum Tragarm 1 dann als Funksignale übermittelt werden und der Handgriff 5 über eine eigene Energieversorgung in Form einer Batterie oder eines Akkus verfügt.

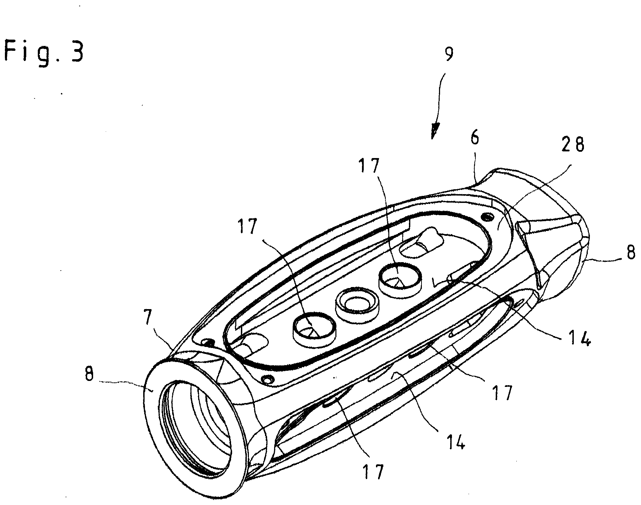

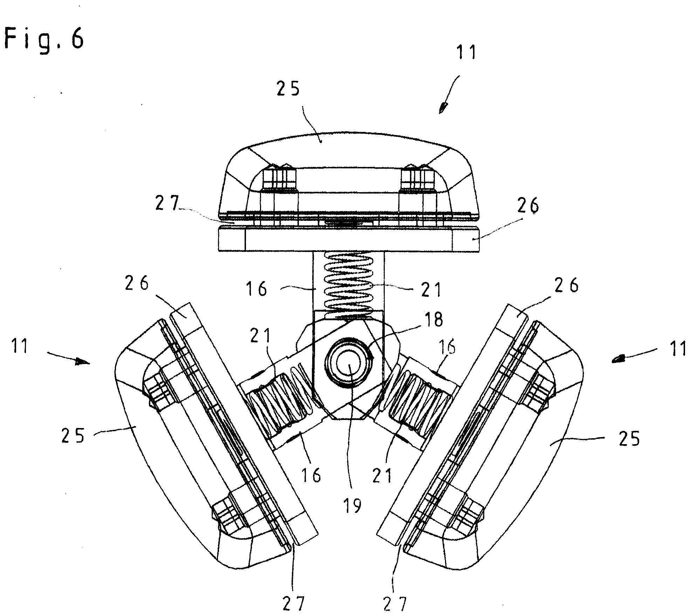

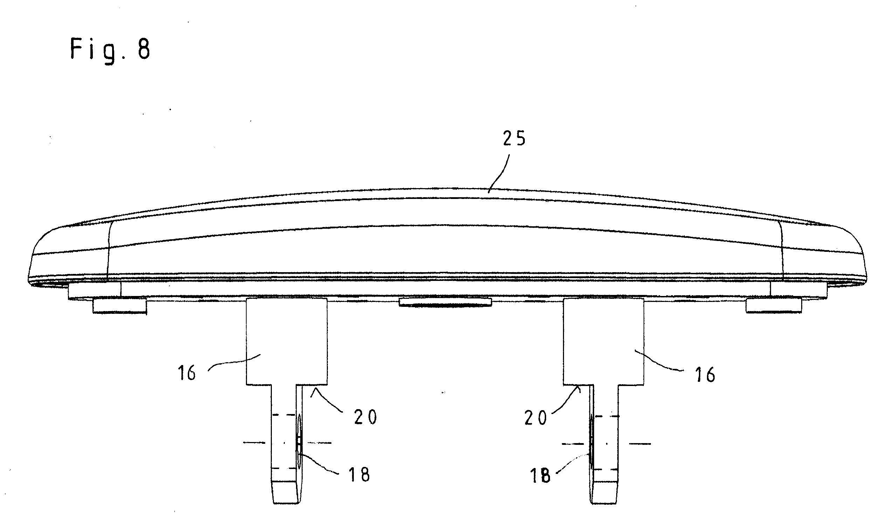

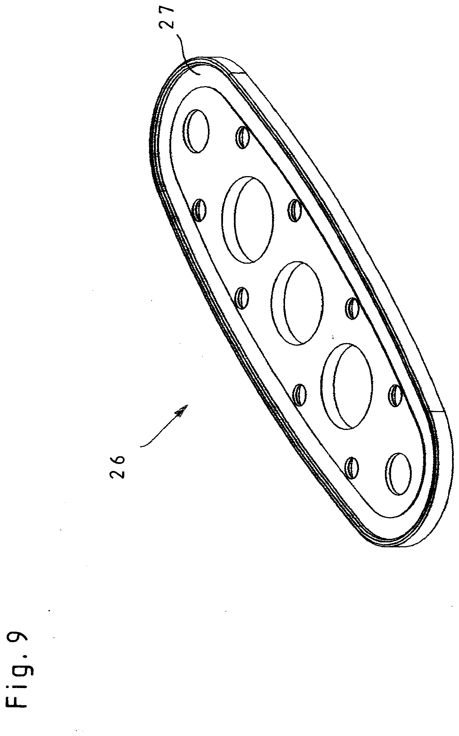

Der Aufbau des Handgriffs 5 wird nachfolgend anhand der Abbildungen Fig. 2 bis 11 detailliert erläutert.

Der Handgriff 5 besteht im Wesentlichen aus einem Handgriffgehäuse 9 in dem mehrere Schaltelemente 11 derart gelagert sind, dass die Schaltelemente 11 federelastisch in das Handgriffgehäuse 9 eindrückbar sind. Um die relativ zum starren Handgriffgehäuse 9 beweglich im Handgriffgehäuse 9 gelagerten Schaltelemente 11 gegenüber dem Handgriffgehäuse 9 abzudichten und so das Autoklavieren des Handgriffs 5 zu ermöglichen, weist jedes Schaltelement 11 ein umlaufendes Dichtungselement 12 aus einem autoklavierbaren Material, wie beispielsweise Silikon, auf, das einerseits am Handgriffgehäuse 9 und andererseits am Schaltelement 11 festgelegt ist.

Wie aus Fig. 2 bis 4 ersichtlich, ist das Handgriffgehäuse 9 des Handgriffs 5 beim dargestellten Ausführungsbeispiel im Querschnitt dreieckig ausgebildet ist und in jeder der drei Seitenflächen 13 des Handgriffgehäuses 9 jeweils ein Schaltelement 11 angeordnet.

Bei der Verwendung des Handgriffs 5 an einem medizinischen Haltearm 1 dient der Handgriff 5 dazu, über die Schaltelemente 11 die Gelenke 4 des Haltearms 1 zu lösen, wenn dieser neu positioniert werden soll. In dem Moment, in dem der Bediener die Schaltelemente 11 betätigt und die Gelenke 4 des Haltearms 1 löst, muss der Bediener das gesamte Gewicht des Haltearms 1 über den Halt am Handgriff 5 auffangen. Durch die Kanten der dreieckigen Form des Handgriffgehäuses 9 und die Kanten der über die Seitenflächen 13 des Handgriffgehäuses 9 herausragenden Schaltelemente 11 bietet der Handgriff 5 einen sicheren Halt und verhindert auch eine Torsion des Handgriffs 5 in der Hand des Bedieners, wie dies ansonsten bei rund geformten Handgriffen 5 passieren kann.

Die Schaltelemente 11 sind in wannenförmigen Vertiefungen 14 des Handgriffgehäuses 9 angeordnet und über an der Unterseite 15 des jeweiligen Schaltelements 11 angeordnete Stege 16 im Handgriffgehäuse 9 gelagert, wozu in der wannenförmigen Vertiefung 14 Öffnungen 17 zum Durchtritt der Stege 16 ausgebildet sind.

Bei dem dargestellten Ausführungsbeispiel ist jedes Schaltelement 11 über jeweils zwei voneinander beabstandete Stege 16 im Handgriffgehäuse 9 gelagert, jedoch ist es selbstverständlich auch möglich, nur einen Steg 16 oder beispielsweise auch drei Stege 16 vorzusehen.

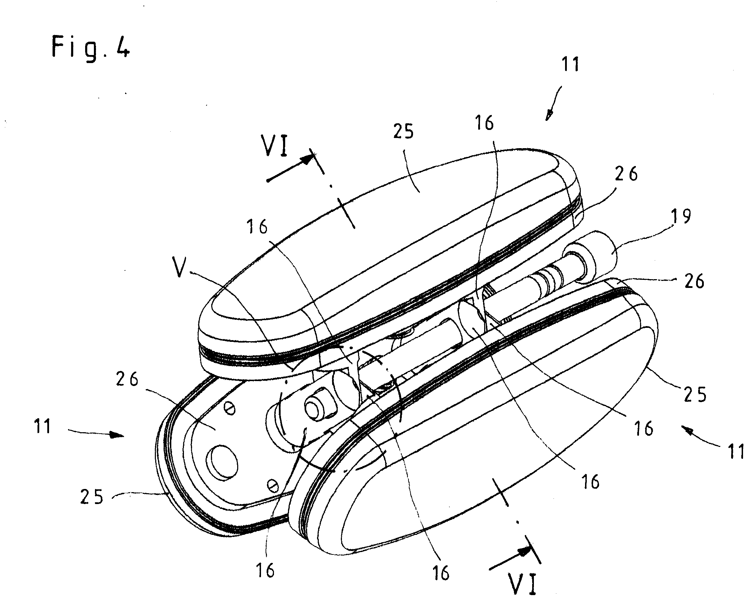

Zur Lagerung der Schaltelemente 11 im Handgriffgehäuse 9 ist in jedem Steg 16 ein in Längsrichtung des jeweiligen Stegs 16 verlaufendes Langloch 18 ausgebildet, das zur Aufnahme einer die Langlöcher 18 aller Stege 16 durchragenden gemeinsamen Achse 19 dient. Die Achse 19 ist im montierten Zustand des Handgriffs 5 ortsfest im Handgriffgehäuse 9 festgelegt. Die Lagerung der Stege 16 auf der Achse 19 ist insbesondere der Abbildung Fig. 4 zu entnehmen.

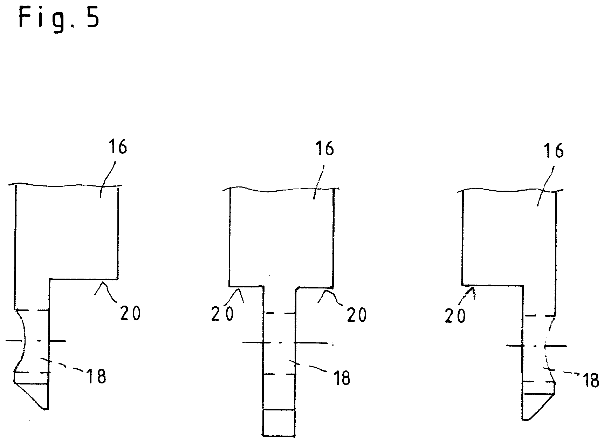

Um zu ermöglichen, dass die vorderen und hinteren Stege 16 der drei Schaltelemente jeweils möglichst kompakt nebeneinander auf der gemeinsamen Achse 19 gelagert sind, weisen die Stege 16 im Bereich der Langlöcher 18 Ausfräsungen 20 auf, wie dies der Abbildung Fig. 5 zu entnehmen ist.

Das federelastische Eindrücken der Schaltelemente 11 in das Handgriffgehäuse 9 erfolgt über jeweils ein Federelement 21, das mit einem Ende an der Unterseite 15 des jeweiligen Schaltelements 11 anliegt und sich mit dem anderen Ende am Boden der wannenförmigen Vertiefung 14 des Handgriffgehäuses 9 abstützt. Vorteilhafterweise ist das Federelement 21 mittig zwischen den beiden Stegen 16 des jeweiligen Schaltelements 11 angeordnet, so dass das Schaltelement 11 nicht nur als Ganzes senkrecht nach unten gedrückt werden kann, sondern auch nach vorne oder nach hinten wippend betätigt werden kann.

Durch die sich an der Bodenplatte der wannenförmigen Vertiefungen 14 abstützenden Federelemente 21 werden die Schaltelemente 11 nach außen gedrückt, bis diese mit den Unterkanten der in den Stegen 16 ausgebildeten Langlöchern 18 an der gemeinsamen Achse 19 anliegen. Somit ergibt sich ein Arbeitshub für die Schaltelemente 11, der der Länge der in den Stegen 16 ausgebildeten Langlöcher 18 entspricht. Diese beschriebene Art der Lagerung der Schaltelemente 11 auf der gemeinsamen Achse 19 gewährleistet einerseits eine sichere und unverlierbare Lagerung der Schaltelemente 11 im Handgriffgehäuse 9 und garantiert andererseits gleichzeitig einen definierten Arbeitshub für die in das Handgriffgehäuse 9 eindrückbaren Schaltelemente 11.

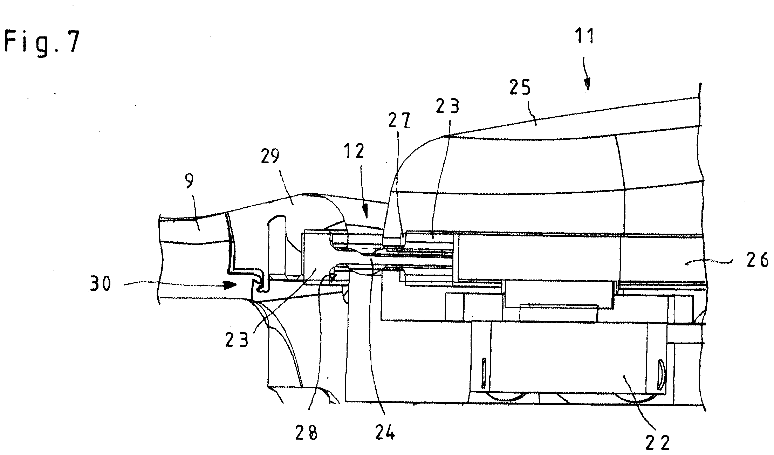

Zum Erzeugen eines Schaltsignals ist unterhalb jedes Schaltelements 11 mindestens ein elektrischer Kontakt 22, beispielsweise ein Taster, so angeordnet ist, dass das Schaltelement 11 den zugehörigen mindestens einen elektrischen Kontakt 22 beim Eindrücken in das Handgriffgehäuse 9 aktiviert. Dieser mindestens eine elektrische Kontakt 22 ist in der wannenförmigen Vertiefung 14 Handgriffgehäuses 9 unterhalb des jeweiligen Schaltelements 11 angeordnet, wie dies in Fig. 7 dargestellt ist.

Vorteilhafterweise sind unterhalb jedes Schaltelements 11 zwei voneinander beabstandete elektrische Kontakte 22 angeordnet sind, wobei die beiden elektrischen Kontakte 22 eines jeden Schaltelements 11 in Parallelschaltung geschaltet sind. Die Verwendung von zwei elektrischen Kontakten ermöglicht auch eine wippende Betätigung des Schaltelements 11 über das Federelement 21. Aufgrund der Parallelschaltung der beiden elektrischen Kontakte 22 ist es für das Erzeugen eines Schaltsignals egal, ob der vordere oder der hintere elektrische Kontakt 22 separat oder gleichzeitig aktiviert werden.

Die zuverlässige Abdichtung zwischen dem Handgriffgehäuse 9 einerseits und dem eindrückbaren Schaltelement 11 andererseits erfolgt über das jedes Schaltelement 11 umgebende umlaufende Dichtungselement 12, wobei das Dichtungselement 12 sowohl auf Seiten des Handgriffgehäuses 9 als auch auf Seiten des jeweiligen Schaltelements 11 klemmend gelagert ist, wie dies der Schnittzeichnung gemäß Fig. 7 zu entnehmen ist. Eine beidseitig klemmende Lagerung des Dichtungselements 12 garantiert einen sicheren und ortsfesten Halt des Dichtungselements 12 auch beim Betätigen des zugehörigen Schaltelements 11.

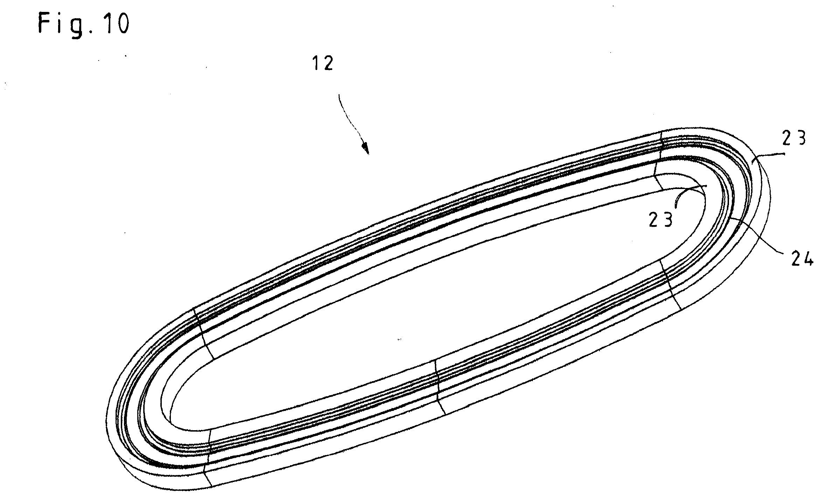

Wie aus den Abbildungen Fig. 7 und 10 ersichtlich, weist jedes Dichtungselement 12 zwei in Radialrichtung voneinander beabstandete wulstförmige Klemmbereiche 23 auf, die über einen elastischen Verbindungssteg 24 miteinander verbunden sind. Der elastische Verbindungssteg 24 gewährleistet das federelastische Eindrücken der Schaltelemente 11 in das Handgriffgehäuse 9 bei gleichzeitig ortfester klemmender Lagerung des Dichtungselement 12 am Handgriffgehäuse 9 und am Schaltelement 11.

Jedes Schaltelement 11 besteht, wie aus einer Zusammenschau der Abbildungen Fig. 7, 8 und 9 ersichtlich, aus einer oberen Griffplatte 25 und einer unteren Schraubplatte 26, wobei das umlaufende Dichtungselement 12 klemmend in einer Dichtungsaufnahme 27 zwischen der Griffplatte 25 und der Schraubplatte 26 festgelegt ist. Die Verwendung des zweiteiligen Aufbaus der Schaltelemente 11 mit der unteren Schraubplatte 26 hat den Vorteil, dass das Dichtungselement 12 auf einfache Art und Weise klemmend an dem jeweilige Schaltelement 11 festlegbar ist und alle notwendigen Schraubverbindungen auf der Unterseite des Schaltelements 11 angeordnet sind, die durch das umlaufende Dichtungselement 12 gegenüber der Umgebung abgedichtet ist. Die üblichen schwer zu reinigenden Stellen, wie beispielsweise Schraubenlöcher, sind somit bei dieser Ausgestaltung konstruktionsbedingt bereits so angeordnet, dass diese von außen nicht zugänglich sind.

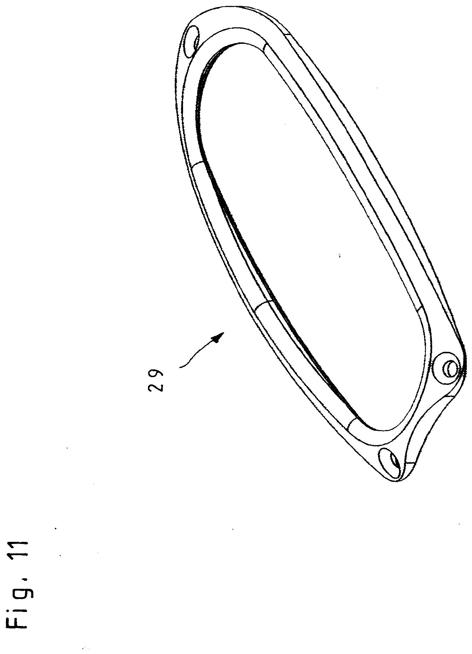

Das klemmende Festlegen des Dichtungselements 12 auf Seiten des Handgriffgehäuses 9 erfolgt dadurch, dass im Handgriffgehäuse 9 umlaufend um jede wannenförmige Vertiefung 14 zur Aufnahme eines Schaltelements 11 eine Nut 28 zur Aufnahme des jeweiligen Dichtungselements 12 ausgebildet ist, wobei die jeweilige Nut 28 bei eingelegtem Dichtungselement 12 über eine am Handgriffgehäuse 9 festlegbare Klemmplatte 29 gemäß Fig. 11 abdeckbar ist. Die Klemmplatte 29 ihrerseits ist über eine Rastverbindung 30 am Handgriffgehäuse 9 festlegbar.

Durch die Verwendung des federelastisch in das Handgriffgehäuse 9 eindrückbaren Schaltelemets 11 mit dem umlaufenden Dichtungselement 12 aus einem autoklavierbaren Material, das den Spalt zwischen dem beweglichen Schaltelement 11 und dem starren Handgriffgehäuse 9 abdichtend überbrückt, wird ein Handgriff 5 geschaffen, der bei einfacher und sicherer Handhabung ohne Fügestellen im Handgriffgehäuse 9 auskommt und daher eine zuverlässige Reinigung im Autoklaven gewährleistet.

Um die Betriebssicherheit des Handgriffs 5 zu erhöhen und insbesondere ein versehentliches Betätigen eines Schaltelements 11 auszuschließen, müssen alle im Handgriffgehäuse 9 angeordneten Schaltelemente 11 zur Erzeugung eines Schaltsignals gleichzeitig betätigt werden. Zu diesem Zweck sind alle im Handgriffgehäuse 9 angeordneten Schaltelemente 11 in Reihe geschaltet sind. Das versehentliche Betätigen nur einzelner Schaltelemente 11 des jeweiligen Handgriffs 5 bleibt somit unschädlich.

Nachfolgend wird kurz die Arbeitsweise zum Betätigen eines wie voranstehend beschrieben aufgebauten Handgriffs 5 erläutert.

Unter jedem Schaltelement 11 befinden sich im konkreten Ausführungsbeispiel zwei elektrische Kontakte 22, nämlich in Längsrichtung des Handgriffgehäuses 9 betrachtet einer an jedem Ende des jeweiligen Schaltelements 11. Diese beiden elektrischen Kontakte 22 des Schaltelements 11 sind parallel geschaltet und wirken somit elektrisch wie ein gemeinsamer Kontakt 22, reagieren aber aufgrund ihrer räumlichen Trennung auch schon dann, wenn das Schaltelement 11 aufgrund der federnden Lagerung auf dem Federelement 21 wippend nur vorne oder nur hinten betätigt wird.

Die elektrischen Kontakte 22 der verschiedenen Schaltelemente 11 eines Handgriffs 5 sind elektrisch in Reihe geschaltet, so dass ein Schaltsignal nur dann erzeugt kann, wenn wirklich alle Schaltelemente 11 gleichzeitig betätigt werden.

Um diese Schaltstellung von Kurzschlüssen auf der Zuleitung unterscheiden zu können, sind parallel und in Serie zu den elektrischen Kontakten 22 Widerstände 31 geschaltet, die nach dem Ruhestrom-Prinzip ausgewertet werden können.

Der Kurzschluss eines einzelnen elektrischen Kontakts 22 kann dadurch erkannt werden, dass der parallele Widerstand durch drei Widerstände 31 mit je einem Drittel Wertigkeit ersetzt wird und diese drei Widerstände 31 zu jeweils nur einem elektrischen Kontakt 22 parallel geschaltet sind. Damit kann eine Auswerteelektronik erkennen, ob keiner, einer, zwei oder drei Schaltelemente 11 gedrückt werden.

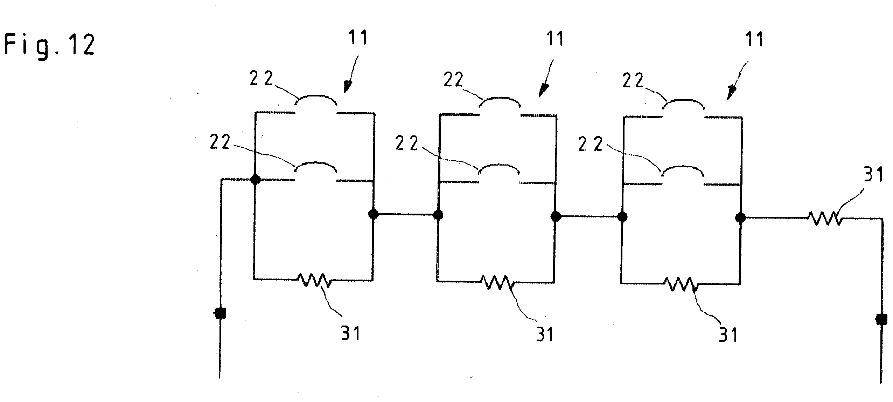

Die Abbildung Fig. 12 zeigt ein schematisches Blockschaltbild der zuvor beschriebenen Schaltung der Schaltelemente 11 des Handgriffs 5.

Wenn ein elektrischer Kontakt 22 einen Kurzschluss-Defekt aufweist, also längere Zeit aktiv ist, ohne dass die anderen Schaltelemente 11 gedrückt werden, kann ein Zeitmessglied dies erkennen und das Gesamtsystem in einen sicheren Zustand überführen. Im Falle des Einsatzes an einem medizinischen Haltearm 1 werden bei der Deaktivierung der Schaltelemente 11 alle Gelenke 4 des Haltearms 1 in der die Gelenke 4 sperrenden Stellung gehalten.

Neben der Autoklavierbarkeit des voranstehend beschriebenen Handgriffs 5 zeichnet sich dieser Handgriff 5 dadurch aus, dass er für den Bediener eine einfache und sichere Handhabung gewährleistet.

### Bezugszeichenliste

- 1: Haltearm
- 2: Basisteil
- 3: Armsegment
- 4: Gelenk
- 5: Handgriff
- 6: proximales Ende
- 7: distales Ende
- 8: Kopplungsanschluss
- 9: Handgriffgehäuse
- 10: elektrisches Kabel
- 11: Schaltelement
- 12: Dichtungselement
- 13: Seitenfläche
- 14: Vertiefung
- 15: Unterseite
- 16: Steg
- 17: Öffnung
- 18: Langloch
- 19: Achse
- 20: Ausfräsung
- 21: Federelement
- 22: elektrischer Kontakt
- 23: Klemmbereich
- 24: Verbindungssteg
- 25: Griffplatte
- 26: Schraubplatte
- 27: Dichtungsaufnahme
- 28: Nut
- 29: Klemmplatte
- 30: Rastverbindung
- 31: Widerstand

## Patentansprüche

1. Autoklavierbarer Handgriff mit einem Handgriffgehäuse (9) und mindestens einem im Handgriffgehäuse (9) gelagerten Schaltelement (11), wobei das mindestens eine Schaltelement (11) federelastisch in das Handgriffgehäuse (9) eindrückbar ist, wobei das Schaltelement (11) über mindestens einen an der Unterseite (15) des Schaltelements (11) angeordneten Steg (16) im Handgriffgehäuse (9) gelagert ist und das Schaltelement (11) gegenüber dem Handgriffgehäuse (9) über ein umlaufendes Dichtungselement (12) abgedichtet ist,
**dadurch gekennzeichnet,**
**dass** das Handgriffgehäuse (9) mehrere in jeweils einer zugehörigen wannenförmigen Vertiefung (14) des Handgriffgehäuses (9) gelagerte Schaltelemente (11) aufweist, wobei in jeder Vertiefung (14) eine Öffnung (17) zum Durchtritt des mindestens einen Stegs (16) ausgebildet ist und wobei für jeden Steg (16) eine separate Öffnung (17) in der wannenförmigen Vertiefung (14) ausgebildet ist und die Stege (16) aller Schaltelemente (11) auf einer gemeinsamen Achse (19) im Handgriffgehäuse (9) gelagert sind.

2. Autoklavierbarer Handgriff nach Anspruch 1, **dadurch gekennzeichnet, dass** in jedem Steg (16) ein in Längsrichtung des Stegs (16) verlaufendes Langloch (18) ausgebildet ist und jeder Steg (16) über eine das Langloch (18) durchragende Achse (19) im Handgriffgehäuse (9) gelagert ist, wobei am Schaltelement (11) ein Federelement (21) derart gelagert ist, dass das Federelement (21) das Schaltelement (11) nach außen aus dem Handgriffgehäuse (9) drückt, so dass sich ein Arbeitsweg des Schaltelements (11) aus der Länge des Langlochs (18) ergibt.

3. Autoklavierbarer Handgriff nach Anspruch 2, **dadurch gekennzeichnet, dass** das Federelement (21) mit einem Ende an der Unterseite (15) des Schaltelements (11) anliegt und sich mit dem anderen Ende am Boden der wannenförmigen Vertiefung (14) des Handgriffgehäuses (9) abstützt.

4. Autoklavierbarer Handgriff nach einem der Ansprüche 1 bis 3, **dadurch gekennzeichnet, dass** jedes Schaltelement (11) über zwei voneinander beabstandete Stege (16) im Handgriffgehäuse (9) gelagert ist, wobei beide Stege (16) auf einer gemeinsamen Achse (19) im Handgriffgehäuse (9) gelagert sind und für jeden Steg (16) eine separate Öffnung (17) in der wannenförmigen Vertiefung (14) des Handgriffgehäuses (9) ausgebildet ist.

5. Autoklavierbarer Handgriff nach einem der Ansprüche 1 bis 4, **dadurch gekennzeichnet, dass** das jeweilige umlaufende Dichtungselement (12), über das jedes Schaltelement (11) gegenüber dem Handgriffgehäuse (9) abgedichtet ist, sowohl auf Seiten des Handgriffgehäuses (9) als auch auf Seiten des jeweiligen Schaltelements (11) klemmend gelagert ist.

6. Autoklavierbarer Handgriff nach Anspruch 5, **dadurch gekennzeichnet, dass** jedes Dichtungselement (12) zwei in Radialrichtung voneinander beabstandete wulstförmige Klemmbereiche (23) aufweist, die über einen elastischen Verbindungssteg (24) miteinander verbunden sind.

7. Autoklavierbarer Handgriff nach Anspruch 5 oder 6, **dadurch gekennzeichnet, dass** jedes Schaltelement (11) aus einer oberen Griffplatte (25) und einer unteren Schraubplatte (26) besteht, wobei das umlaufende Dichtungselement (12) klemmend in einer Dichtungsaufnahme (27) zwischen der Griffplatte (25) und der Schraubplatte (26) festgelegt ist.

8. Autoklavierbarer Handgriff nach einem der Ansprüche 5 bis 7, **dadurch gekennzeichnet, dass** im Handgriffgehäuse (9) umlaufend um jede wannenförmige Vertiefung (14) zur Aufnahme eines Schaltelements (11) eine Nut (28) zur Aufnahme des jeweiligen Dichtungselements (12) ausgebildet ist, wobei die jeweilige Nut (28) bei eingelegtem Dichtungselement (12) über eine am Handgriffgehäuse (9) festlegbare Klemmplatte (29) abdeckbar ist.

9. Autoklavierbarer Handgriff nach Anspruch 8, **dadurch gekennzeichnet, dass** die Klemmplatte (29) über eine Rastverbindung (30) am Handgriffgehäuse (9) festlegbar ist.

10. Autoklavierbarer Handgriff nach einem der Ansprüche 1 bis 9, **dadurch gekennzeichnet, dass** das Handgriffgehäuse (9) im Querschnitt dreieckig ausgebildet ist und, dass in jeder der drei Seitenflächen (13) des Handgriffgehäuses (9) jeweils eine wannenförmige Vertiefung (14) ausgebildet ist, in der ein Schaltelement (11) federelastisch in das Handgriffgehäuse (9) eindrückbar gelagert ist.

11. Autoklavierbarer Handgriff nach einem der Ansprüche 1 bis 10, **dadurch gekennzeichnet, dass** unterhalb jedes Schaltelements (11) mindestens ein elektrischer Kontakt (22) so angeordnet ist, dass das Schaltelement (11) den zugehörigen mindestens einen elektrischen Kontakt (22) beim Eindrücken in das Handgriffgehäuse (9) aktiviert.

12. Autoklavierbarer Handgriff nach Anspruch 11, **dadurch gekennzeichnet, dass** der mindestens eine elektrische Kontakt (22) in der wannenförmigen Vertiefung (14) des Handgriffgehäuses (9) angeordnet ist.

13. Autoklavierbarer Handgriff nach Anspruch 11 oder 12, **dadurch gekennzeichnet, dass** unterhalb jedes Schaltelements (11) zwei elektrische Kontakte (22) angeordnet sind, wobei die beiden elektrischen Kontakte (22) eines jeden Schaltelements (11) in Parallelschaltung geschaltet sind.

14. Verfahren zum Betätigen der Schaltelemente eines autoklavierbaren Handgriffs nach einem der Ansprüche 1 bis 13, wobei im Handgriffgehäuse (9) mindestens zwei federelastisch in das Handgriffgehäuse (9) eindrückbare Schaltelemente (11) gelagert sind,
**dadurch gekennzeichnet,**
**dass** alle im Handgriffgehäuse (9) angeordneten Schaltelemente (11) zur Erzeugung eines Schaltsignals gleichzeitig betätigt werden.

15. Verfahren nach Anspruch 14, **dadurch gekennzeichnet, dass** alle im Handgriffgehäuse (9) angeordneten Schaltelemente (11) in Reihe geschaltet sind.

16. Verfahren nach Anspruch 14 oder 15, **dadurch gekennzeichnet, dass** jedes Schaltelement (11) mittels einer nach dem Ruhestromprinzip arbeitenden Sicherheitsschaltung überwacht wird.

17. Verfahren nach einem der Ansprüche 14 bis 16, **dadurch gekennzeichnet, dass** alle Schaltelemente (11) eines Handgriffs (5) mittels eines Zeitmessglieds auf das Vorliegen eines Kurzschluss-Defekts derart überwacht werden, dass beim Ausfall eines Schaltelements (11) alle anderen Schaltelemente (11) dieses Handgriffs (5) deaktiviert werden.

## Claims

1. Autoclavable handle with a handle housing (9) and at least one switching element (11) which is mounted in the handle housing (9), it being possible for the at least one switching element (11) to be pressed in a resilient manner into the handle housing (9), the switching element (11) being mounted in the handle housing (9) via at least one web (16) which is arranged on the underside (15) of the switching element (11), and the switching element (11) being sealed with respect to the handle housing (9) via a peripheral seal element (12), **characterized in that** the handle housing (9) has a plurality of switching elements (11) which are mounted in in each case one associated trough-shaped depression (14) of the handle housing (9), an opening (17) for the passage of the at least one web (16) being configured in each depression (14), and a separate opening (17) being configured in the trough-shaped depression (14) for each web (16), and the webs (16) of all the switching elements (11) being mounted on a common axle (19) in the handle housing (9).

2. Autoclavable handle according to Claim 1, **characterized in that** a slot (18) which runs in the longitudinal direction of the web (16) is configured in each web (16), and each web (16) is mounted in the handle housing (9) via an axle (19) which protrudes through the slot (18), a spring element (21) being mounted on the switching element (11) in such a way that the spring element (21) presses the switching element (11) to the outside out of the handle housing (9), with the result that an operating travel of the switching element (11) arises from the length of the slot (18).

3. Autoclavable handle according to Claim 2, **characterized in that** the spring element (21) bears with one end against the underside (15) of the switching element (11), and is supported with the other end on the bottom of the trough-shaped depression (14) of the handle housing (9).

4. Autoclavable handle according to one of Claims 1 to 3, **characterized in that** each switching element (11) is mounted in the handle housing (9) via two webs (16) which are spaced apart from one another, the two webs (16) being mounted in the handle housing (9) on a common axle (19), and a separate opening (17) being configured in the trough-shaped depression (14) of the handle housing (9) for each web (16).

5. Autoclavable handle according to one of Claims 1 to 4, **characterized in that** the respective peripheral seal element (12), via which each switching element (11) is sealed with respect to the handle housing (9), is mounted in a clamping manner both on the side of the handle housing (9) and on the side of the respective switching element (11).

6. Autoclavable handle according to Claim 5, **characterized in that** each seal element (12) has two bead-shaped clamping regions (23) which are spaced apart from one another in the radial direction and are connected to one another via an elastic connecting, web (24).

7. Autoclavable handle according to Claim 5 or 6, **characterized in that** each switching element (11) consists of an upper handle plate (25) and a lower screwing plate (26), the peripheral seal element (12) being fixed in a clamping manner in a seal seat (27) between the handle plate (25) and the screwing plate (26) .

8. Autoclavable handle according to one of Claims 5 to 7, **characterized in that** a groove (28) for receiving the respective seal element (12) is configured in the handle housing (9) in a peripheral manner around each trough-shaped depression (14) for receiving a switching element (11), it being possible, in the case of an inserted seal element (12), for the respective groove (28) to be covered via a clamping plate (29) which can be fixed on the handle housing (9).

9. Autoclavable handle according to Claim 8, **characterized in that** the clamping plate (29) can be fixed on the handle housing (9) via a latching connection (30).

10. Autoclavable handle according to one of Claims 1 to 9, **characterized in that** the handle housing (9) is of triangular configuration in cross section, and **in that** in each case one trough-shaped depression (14) is configured in each of the three side faces (13) of the handle housing (9), in which trough-shaped depression (14) a switching element (11) can be pressed in a resilient manner into the handle housing (9).

11. Autoclavable handle according to one of Claims 1 to 10, **characterized in that** at least one electric contact (22) is arranged below each switching element (11) in such a way that the switching element (11) activates the associated at least one electric contact (22) in the case of being pressed into the handle housing (9).

12. Autoclavable handle according to Claim 11, **characterized in that** the at least one electric contact (22) is arranged in the trough-shaped depression (14) of the handle housing (9).

13. Autoclavable handle according to Claim 11 or 12, **characterized in that** two electric contacts (22) are arranged below each switching element (11), the two electric contacts (22) of each switching element (11) being connected in a parallel circuit.

14. Method for actuating the switching elements of an autoclavable handle according to one of Claims 1 to 13, at least two switching elements (11) which can be pressed in a resilient manner into the handle housing (9) being mounted in the handle housing (9), **characterized in that** all the switching elements (11) which are arranged in the handle housing (9) are actuated at the same time in order to generate a switching signal.

15. Method according to Claim 14, **characterized in that** all the switching elements (11) which are arranged in the handle housing (9) are connected in series.

16. Method according to Claim 14 or 15, **characterized in that** each switching element (11) is monitored by means of a safety circuit which operates in accordance with the closed current principle.

17. Method according to one of Claims 14 to 16, **characterized in that** all the switching elements (11) of a handle (5) are monitored for the presence of a short circuit fault by means of a timer in such a way that, in the case of the failure of one switching element (11), all the other switching elements (11) of the said handle (5) are deactivated.

## Revendications

1. Poignée pouvant être autoclavée, comportant un boîtier de poignée (9) et au moins un élément de commutation (11) monté dans le boîtier de poignée (9), dans lequel l'au moins un élément de commutation (11) peut être enfoncé de manière élastique dans le boîtier de poignée (9), dans lequel l'élément de commutation (11) est monté dans le boîtier de poignée (9) par le biais d'au moins une nervure (16) disposée au niveau du côté inférieur (15) de l'élément de commutation (11) et l'élément de commutation (11) est fermé hermétiquement par rapport au boîtier de poignée (9) par le biais d'un élément d'étanchéité périphérique (12),
**caractérisée en ce que**
le boîtier de poignée (9) comprend plusieurs éléments de commutation (11) montés respectivement dans un évidement (14) en forme de bac associé du boîtier de poignée (9), une ouverture (17) pour le passage de l'au moins une nervure (16) étant réalisée dans chaque évidement (14), et une ouverture séparée (17) étant réalisée dans l'évidement (14) en forme de bac pour chaque nervure (16) et les nervures (16) de tous les éléments de commutation (11) étant montées sur un axe commun (19) dans le boîtier de poignée (9).

2. Poignée pouvant être autoclavée selon la revendication 1, **caractérisée en ce qu'**un trou oblong (18) s'étendant dans la direction longitudinale de la nervure (16) est réalisé dans chaque nervure (16), et chaque nervure (16) est montée dans le boîtier de poignée (9) par le biais d'un axe (19) traversant le trou oblong (18), un élément ressort (21) étant monté sur l'élément de commutation (11) de telle sorte que l'élément ressort (21) presse l'élément de commutation (11) vers l'extérieur à partir du boîtier de poignée (9), de sorte qu'une course de travail de l'élément de commutation (11) résulte de la longueur du trou oblong (18) .

3. Poignée pouvant être autoclavée selon la revendication 2, **caractérisée en ce que** l'élément ressort (21) s'appuie, par une extrémité, contre le côté inférieur (15) de l'élément de commutation (11) et est supporté, par l'autre extrémité, sur le fond de l'évidement (14) en forme de bac du boîtier de poignée (9) .

4. Poignée pouvant être autoclavée selon l'une des revendications 1 à 3, **caractérisée en ce que** chaque élément de commutation (11) est monté dans le boîtier de poignée (9) par le biais de deux nervures (16) espacées l'une de l'autre, les deux nervures (16) étant montées dans le boîtier de poignée (9) sur un axe commun (19) et une ouverture séparée (17) étant réalisée dans l'évidement (14) en forme de bac du boîtier de poignée (9) pour chaque nervure (16).

5. Poignée pouvant être autoclavée selon l'une des revendications 1 à 4, **caractérisée en ce que** l'élément d'étanchéité périphérique respectif (12), par le biais duquel chaque élément de commutation (11) est fermé hermétiquement par rapport au boîtier de poignée (9), est monté de manière serrée à la fois sur des côtés du boîtier de poignée (9) et sur des côtés de l'élément de commutation respectif (11).

6. Poignée pouvant être autoclavée selon la revendication 5, **caractérisée en ce que** chaque élément d'étanchéité (12) comprend deux régions de serrage (23) en forme de bourrelets espacées l'une de l'autre dans la direction radiale, lesquelles sont reliées l'une à l'autre par le biais d'une nervure de liaison élastique (24).

7. Poignée pouvant être autoclavée selon la revendication 5 ou 6, **caractérisée en ce que** chaque élément de commutation (11) est constitué d'une plaque de préhension supérieure (25) et d'une plaque à visser inférieure (26), l'élément d'étanchéité périphérique (12) étant fixé de manière serrée dans un logement d'étanchéité (27) entre la plaque de préhension (25) et la plaque à visser (26).

8. Poignée pouvant être autoclavée selon l'une des revendications 5 à 7, **caractérisée en ce qu'**une rainure (28) destinée à loger l'élément d'étanchéité respectif (12) est réalisée dans le boîtier de poignée (9) de manière périphérique autour de chaque évidement (14) en forme de bac servant au logement d'un élément de commutation (11), la rainure respective (28) étant recouverte par le biais d'une plaque de serrage (29) pouvant être fixée au boîtier de poignée (9) lorsque l'élément d'étanchéité (12) est introduit.

9. Poignée pouvant être autoclavée selon la revendication 8, **caractérisée en ce que** la plaque de serrage (29) peut être fixée au boîtier de poignée (9) par le biais d'une liaison par encliquetage (30).

10. Poignée pouvant être autoclavée selon l'une des revendications 1 à 9, **caractérisée en ce que** le boîtier de poignée (9) est réalisé de manière triangulaire en section transversale, et **en ce qu'**un évidement (14) en forme de bac est réalisé respectivement dans chacune des trois surfaces latérales (13) du boîtier de poignée (9), évidement dans lequel un élément de commutation (11) peut être enfoncé de manière élastique dans le boîtier de poignée (9).

11. Poignée pouvant être autoclavée selon l'une des revendications 1 à 10, **caractérisée en ce qu'**au moins un contact électrique (22) est disposé en dessous de chaque élément de commutation (11), de telle sorte que l'élément de commutation (11) active l'au moins un contact électrique (22) associé lors de l'enfoncement dans le boîtier de poignée (9).

12. Poignée pouvant être autoclavée selon la revendication 11, **caractérisée en ce que** l'au moins un contact électrique (22) est disposé dans l'évidement (14) en forme de bac du boîtier de poignée (9).

13. Poignée pouvant être autoclavée selon la revendication 11 ou 12, **caractérisée en ce que** deux contacts électriques (22) sont disposés en dessous de chaque élément de commutation (11), les deux contacts électriques (22) de chaque élément de commutation (11) étant montés suivant un montage en parallèle.

14. Procédé permettant d'actionner les éléments de commutation d'une poignée pouvant être autoclavée selon l'une des revendications 1 à 13, dans lequel au moins deux éléments de commutation (11) pouvant être enfoncés de manière élastique dans le boîtier de poignée (9) sont montés dans le boîtier de poignée (9),
**caractérisé en ce que**
tous les éléments de commutation (11) disposés dans le boîtier de poignée (9) sont actionnés simultanément pour la génération d'un signal de commutation.

15. Procédé selon la revendication 14, **caractérisé en ce que** tous les éléments de commutation (11) disposés dans le boîtier de poignée (9) sont montés en série.

16. Procédé selon la revendication 14 ou 15, **caractérisé en ce que** chaque élément de commutation (11) est surveillé au moyen d'un circuit de sécurité fonctionnant suivant le principe du courant de repos.

17. Procédé selon l'une des revendications 14 à 16, **caractérisé en ce que** tous les éléments de commutation (11) d'une poignée (5) sont surveillés au moyen d'un organe de mesure de temps pour détecter la présence d'un défaut de type court-circuit, de telle sorte qu'en cas de défaillance d'un élément de commutation (11), tous les autres éléments de commutation (11) de cette poignée (5) sont désactivés.
